# EUROPEAN PATENT APPLICATION

(11) **EP 3 903 811 A1**
(43) Date of publication of application: **03.11.2021**
(21) Application number: 20172623.9
(22) Date of filing: 01.05.2020
(51) Int. Cl.: A61K 39/00, A61K 39/12, A61P 31/14, A61P 35/00

(54) **PHARMACEUTICAL COMPOSITION AND METHOD FOR INDUCING AN IMMUNE RESPONSE**

(71) Applicant: R.G.C.C. Holdings AG, 6301 Zug (CH)
(72) Inventor: PAPASOTIRIOU, Ioannis, 6314 Unteraegeri (CH)
(74) Representative: Pestalozzi, Deborah

(57) **Abstract**

The present invention relates to a method for inducing an immune response in a human or animal subject, as well as to a pharmaceutical composition for inducing an immune response, furthermore to a method for producing the pharmaceutical composition in vitro and the use of cytotoxic CD8+ T-lymphocytes activated to recognize an antigenic peptide in a pharmaceutical composition or in a method for inducing an immune response.

## Description

### TECHNICAL FIELD

The present invention relates to the field of cellular immunology and immunotherapy. More specifically, the present invention relates to a method for inducing an immune response in a human or animal patient, as well as to a pharmaceutical composition for inducing an immune response, furthermore to a method for producing the pharmaceutical composition in vitro and the use of primed dendritic cells or of activated cytotoxic CD8+ T-lymphocytes in a pharmaceutical composition or in a method for inducing an immune response.

### PRIOR ART

Pathogens, such as viruses, bacteria, fungi and parasites are organisms that can cause a disease, while some pathogens have be found to be responsible for severe effects and casualties in afflicted hosts.
Vaccination or administration of antibiotics can be useful in preventing or fighting disease arising from pathogens. Cancer is usually treated by surgical resection of tumors, or with aggressive treatments such as chemotherapy or radiotherapy. Those therapies have succeeded in minimizing or inhibiting metastasis and proliferation of cancer cells, but not in effectively defeating cancers. However, due to severe toxicities and recurrence of tumors the life of the patient might still be at risk.

The immune system of the human body provides defense against some common pathogens. Pathogens comprise proteins, so-called antigens that can be recognized by the immune system of the host. Similarly, immune cells, recognize tumor-associated natural peptide antigens presented on the cell surface of tumor cells. Those antigens include proteins that are overexpressed, abnormally expressed embryonic proteins, products from mutated suppressor genes or oncogenes, or derived from cancer-causing viruses in cancer cells.
Antigens can belong to many different chemical classes and can derive from viral or bacterial proteins, lipids, carbohydrates, or combinations of these, such as lipoproteins or glycoproteins. Antigens can also be small chemical compounds, termed haptens, made synthetically in the laboratory, such as nitrophenyl (NP), or be a natural compound introduced into the host, such as urushiol, the toxin found in poison ivy, which becomes modified and antigenic when introduced into the host. Haptens generally require linkage to a larger host protein or foreign protein to become immunogenic.

Cytotoxic T lymphocytes (CTL) are white blood cells that have the ability to kill other cells of the body in a highly specific manner. CTLs are CD8+ T cells that have been stimulated by peptides presented by the MHC I (major histocompatibility complex class I) on affected cells. When CTL have been stimulated by antigen, they migrate through the tissues of the body to find and kill the target cells that are bearing the specific antigen. Antigen-specific CTLs proliferate to produce daughter cells with the same antigen specificity as the parent cells. The total number of those antigen-specific CTLs in the body is increased by the cell division of the activated CTLs.

Dendritic cells (DCs) provide the signals that are required for the activation of T cells and they are potent antigen-presenting cells (APC) in the immune system. Interaction between the antigen presented by a major histocompatibility complex class I or II protein (MHC I/II) that is present on the APCs and the T-cell receptor/CD3 complex is responsible for the specificity of the immune response. This interaction is necessary for T cell activation, but not sufficient. Interaction between receptor-ligand pairs of APCs and T cells generates costimulatory signals that can lead to induction of effector T cell functions and to the full proliferation of T cells.

T cells have the antigen-specific receptor, TCR, that recognizes a physical complex between host MHC proteins and small peptide fragments derived from protein antigens. The interaction between the peptide and MHC molecule is highly specific. MHC class I molecules present peptide antigens to CD8+ T cells and the size of those peptides that can be bound is 8 to 10 amino acids.

Immune recognition of pathogen- or cancer-associated antigens is performed by specific CD8+ cytotoxic T lymphocytes that interact with the peptides that are bound to MHC I molecules. The in vitro stimulation of that interaction can be performed with the presentation of those molecules by APCs and especially the DCs. "Priming" or "pulsing" is the in vitro step, in which dendritic cells first contact the antigen and are then "loaded" with the respective antigen, i.e. present the antigenic peptide on their MHC I molecules. This is an essential step in the subsequent antigen presentation to the CD4+ or CD8+ T cells, i.e. T cell activation. CD8+ T cells that have been activated by the APCs (said activated CD8+ T cells are termed CTLs in the scope of this application) can recognize the same MHC/peptide complex on the target cells, e.g. pathogen-infected or cancer cells, and be triggered to kill them.

Immunotherapy therefore activates the patient's own immune system to recognize and kill the cells presenting antigens, whether pathogen-derived or cancer-derived. Immunotherapy has become a promising alternative in the treatment of cancer patients. The toxic effects of that type of therapy are limited compared to chemotherapy or radiotherapy.

Adoptive cell therapy is the ex vivo activation and expansion of immune effector cells and the administration for the treatment and/or prevention of a disease. The development of a successful strategy for treating a human disease requires an understanding of the responses of the immune cells that participate in the control of the pathogenic condition. The immune cells can be nonspecific effector cells, such as natural killer cells and macrophages, effector cells with limited diversity for antigen recognition, like γδ T cells, and highly specific effector cells that have enormous diversity in antigen recognition such as antibody-producing B cells and αβ T cells. Adoptive cell therapy encompasses the use of antigen-reactive T cells derived from an endogenous source (tumor-specific T-cells, such as tumor infiltrating lymphocytes or from peripheral blood) or a genetically modified population engineered to recognize antigen through expression of the cognate chimeric antibody or T cell receptor (CAR/TCR).

Cancer cells have mechanisms that help them to escape the immune system. Several approaches are used in order to enhance the efficacy of immunotherapy and stop the immune evasion of cancer cells.
Immune checkpoint blockades with monoclonal antibodies that block programmed cell death-1 (PD-1), programmed death ligand-1 (PD-L1) or cytotoxic T lymphocyte-associated protein-4 (CTLA-4), tumor cell vaccines, dendritic cell vaccines and adoptive cell therapy (ACT) like the chimeric antigen receptor (CAR) T cells have shown that immunotherapy has the potential to be used as a basic therapeutic option in many cancers [Farkona S., et al., BMC Med. 14, 73 (2016)].
While immune checkpoint inhibitors are used in order to boost the patient's pre-existing T cell population, they are likely to fail especially in poorly immunogenic cancer types. Administration of tumor-specific T cells by adoptive cell therapy may enable immune system to overcome issues of poor immunogenicity. The recognition of tumor associated antigens (TAAs) is granted by the diverse receptor that allows identification of a variety of peptides upon presentation by MHC molecules. However, the TCR-derived signal via the engagement with peptide/MHC complexes alone is not able to trigger an adequate T cell activation, as the proliferation, differentiation and survival of T cells also require signals from costimulatory molecules such as CD28 and its ligands CD80 and CD86.

Epitope identification often involves derivation and testing of overlapping peptide libraries from the pathogen or tumor-derived proteins that are based on known protein databases. Development and refinement of algorithms that predict pathogen-associated epitopes as well as the definition of preferred peptide-binding characteristics for MHC proteins that are associated with susceptibility to autoimmune disease or infection has been an important tool for the selection of epitopes with high immunogenicity.

The challenge has been the administration of an antigen to induce a CTL response and keep it over time. In vitro, MHC I can be loaded externally (ex vivo, in vitro) with a synthetic peptide to elicit CTL response, such as disclosed e.g. in EP1448229A2.

However, usually the amount of loaded dendritic cells administered however, can be insufficient to activate a sufficient amount of CD8+ T cells in vivo or the immunosuppressive tumor microenvironment may prevent DCs from effectively activating CD8+ T cells to eliminate the tumor in vivo. The ex vivo activation of CD8+ T cells is followed by a determination of the functionality and cytotoxicity of the activated CTL and then the administration of the pharmaceutical composition comprising the CTL as an autologous "living drug" with a specific target into the patient for the purpose of inducing an enhanced immune response in vivo.

### SUMMARY OF THE INVENTION

An object of the present invention therefore is to provide an improved method for adoptive, autologous cell therapy by priming of dendritic cells ex vivo for subsequent administration back to the patient for the triggering of a strong immune response in vivo, or for subsequent ex vivo activation and expansion of patient-derived immune effector cells and the administration of the ex vivo activated CTL back to the patient for the triggering of a strong immune response in vivo.

The present invention relates in a first aspect to an ex vivo/in vitro method for the generation and expansion of patient-derived antigen-specific cytotoxic T lymphocytes (activated CD8+ T cells, i.e. CTL) suitable for administration back to the patient for autologous endogenous adoptive T cell therapy. The method comprises co-culturing of CD8+ T cells from a patient with dendritic cells that have been loaded with a single MHC class I 9-mer or 16-mer peptide specific for the patient's disease type and with other types of immune cells including other subtypes of T lymphocytes, NK cells, B lymphocytes, neutrophils, basophils, eosinophils and platelets. Moreover, the invention relates to the activated, i.e. antigen-specific CD8+ T cells (CTL) obtained by the method and uses thereof.
In a second aspect, the present invention relates to an ex vivo method for stimulation of dendritic cells, i.e. priming (pulsing, loading) in the presence of an antigenic peptide, preferably a viral antigenic peptide, resulting in a population of antigen-presenting dendritic cells (DCs) which can be included in a pharmaceutical composition as a vaccine for administration to a human or animal subject for the purpose of preventing a pathogenic, preferably viral infection by a specific pathogen or virus, or for boosting the immune system of a human or animal subject already infected by a specific pathogen, preferably virus.

More specifically, the invention is related, with respect to the first aspect of the invention, to a pharmaceutical composition for inducing an immune response in a human or animal subject suffering from a pathologic disease or disorder, comprising autologous activated cytotoxic CD8+ T cells, i.e. activated cytotoxic CD8+ T lymphocytes (termed CTLs). The CTLs of said pharmaceutical composition have been activated by autologous, primed mature dendritic cells (DCs) of the human or animal subject, wherein said autologous primed mature dendritic cells present an antigenic peptide. The term "primed", or "pulsed" or "loaded", respectively, means that the autologous dendritic cells, in a still immature state, have been contacted with an antigenic peptide, resulting in "loaded" mature dendritic cells, which present the antigenic peptide on their cell surface MHC I proteins.
In said pharmaceutical composition, the activated CTLs are derived as CD8+ T cells from an autologous population of peripheral blood mononuclear cells (PBMCs) isolated from peripheral blood of the same human or animal subject. Once activated, the CTLs are able to recognize the antigenic peptide. The CTLs have been activated by the autologous DCs primed/pulsed/loaded with an antigenic peptide, wherein the DC have been isolated from the same human or animal subject as the CD8+ T cells which were activated to CTLs. Either the DC are isolated from a separate sample of peripheral blood of the same human or animal subject or patient. However, preferably, in order to minimize the number of invasive interventions, the DC are derived from the same sample of peripheral blood, i.e. the same population of PBMCs as the CD8+ T cells. The CTLs contained in the pharmaceutical composition have been activated by co-culturing T-cells derived from the population of PBMC, with the antigen-presenting dendritic cells. However, preferably, not only T-cells are co-cultured with the loaded mature DCs, but also other non-adherent immune cells. Preferably, the CTLs serving as an active ingredient in the pharmaceutical composition have been activated by co-culturing with the loaded mature DC not only the CD8+ T cells and/or the CD4+ T cells, but by co-culturing with the loaded DC a population of non-adhering immune cells derived from the same population of PBMC as the population of which the dendritic cells have been derived. Said population of non-adherent immune cells include the CD8+ T cells to be activated, as well as at least one of the following group of cells: CD4+ T-cells, NK cells, B lymphocytes, platelets, neutrophils, basophils and eosinophils. More preferably, the CTLs have been activated by co-culturing with the loaded mature DC a population of all non-adhering immune cells derived from the same population of PBMC as the population of which the dendritic cells have been derived.
This is especially advantageous, because mature DCs differentiate further, a process called licensing, when they have signals from other lymphocytes, such as CD4+ T cells, Natural killer cells (NK) and natural killer T-cells (NKT), and they can induce long-lived memory CD8+ T cells. Those signals have various effects on DCs, including secretion of cytokines and upregulation of costimulatory and anti-apoptotic molecules, resulting in the ability of DCs to activate T cells, especially CD8+ T cells. Moreover, there can be a direct effect of the signals of helper lymphocytes on the development of effector T cells, differentiation to effector or memory cells, or on cell cycle progression and cell survival.
Prior to their use in the activation of CD8+ T cells, the DCs have been cultured ex vivo, by culturing monocytes isolated from the population of PBMCs preferably in a growth medium with granulocyte-monocyte colony-stimulating factor and IL-4, resulting in a population of immature DCs. Subsequently, the immature DCs have been primed/pulsed by exposing them to the antigenic peptide, resulting in a population of DC loaded with the antigenic peptide, and have been matured in the presence of a cytokine cocktail, resulting in a population of loaded mature DCs, prior to their use in activation of the CD8+ CTLs by co-culturing as mentioned above.

With respect to the second aspect of the invention, the present invention relates to a pharmaceutical composition for inducing an immune response in a human or animal subject suffering from a pathologic disease or disorder, comprising autologous primed DCs presenting an antigenic peptide on their MHC. This pharmaceutical composition can then be administered to the human or animal subject for inducing an immune response in vivo. This pharmaceutical composition can be used as a vaccine for a human or animal subject, or, in case the subject has already been infected by the pathogen, e.g. virus, for which the antigenic peptide presented on the DCs is specific, as a medicament for treating the human or animal subject.

According to a preferred embodiment of the present invention, the antigenic peptide used for the priming of the DCs and for the subsequent ex vivo or in vivo activation of the CD8+ T cells, the activated CTLs are capable of recognizing after their activation ex vivo or in vivo, is a tumor antigenic peptide, preferably a MUC1-peptide, more preferably of a length of at least 9 amino acids, preferably of exactly 9 amino acids, and most preferably is a MUC (79-87) TLAPATEPA peptide (Seq. ID 1).

In a further preferred embodiment, the antigenic peptide is a viral antigenic peptide, preferably a peptide of SARS-CoV2 (the Severe Acute Respiratory Syndrome Coronavirus 2) S-protein, the so-called "Spike-protein". Preferably, in case of a viral antigenic peptide, the antigenic peptide presented on the loaded mature DCs and used for the activation of the CD8+ CTLs is either a SARS-CoV2 S-protein (84-92) LPFNDGVYF peptide (Seq. ID 2), a SARS-CoV2 S-protein (1185-1200) RLNEVAKNLNESLIDL peptide (Seq. ID 3), a SARS-CoV2 S-protein (1185-1193) RLNEVAKNL peptide (Seq. ID 4), or a SARS-CoV2 S-protein (1192-1200) NLNESLIDL peptide (Seq. ID 5).

The present invention further comprises a method for obtaining human or animal autologous dendritic cells presenting an antigenic peptide, i.e. loaded with said antigenic peptide, preferably a tumor antigenic peptide or viral antigenic peptide of a length of at least 9 amino acids, more preferably selected from the following group consisting of MUC(79-87) TLAPATEPA peptide, SARS-CoV2 S-protein (84-92) LPFNDGVYF peptide (Seq. ID 2), SARS-CoV2 S-protein (1185-1200) RLNEVAKNLNESLIDL peptide (Seq. ID 3), SARS-CoV2 S-protein (1185-1193) RLNEVAKNL peptide (Seq. ID 4), and SARS-CoV2 S-protein (1192-1200) NLNESLIDL peptide (Seq. ID 5), for the preparation of a pharmaceutical composition as described above, comprising the following steps:
a.) culturing monocytes isolated from peripheral blood mononuclear cells (PBMC) of the human or animal subject suffering from a pathologic disease or disorder, preferably suffering from cancer or a viral infection, said monocytes preferably isolated with Ficoll-separation;
b.) culturing of adhering monocytes of step a.) with granulocyte-monocyte colony-stimulating factor and IL-4, preferably in RPMI 1640 Medium with 10% heat-inactivated FBS and 1% glutamine, preferably for 6 days, resulting in a population of immature dendritic cells;
c.) pulsing of the immature dendritic cells of step b.), preferably at day 6 of culture with the antigenic peptide, preferably at a final concentration of 10 µg/ml of the antigenic peptide, and incubation, preferably for 5h, preferably in the presence of β2 microglobulin, preferably at a final concentration of 3µg/ml of β2 microglobulin, resulting in a population of loaded, but still immature DCs presenting the antigenic peptide on their cell surface MHC I protein complexes;
d.) maturing of the loaded DCs presenting the antigenic peptide of step c.) with a cytokine cocktail, preferably including IL-6, preferably IL-6 at a concentration of 10ng/ml, IL-1β, preferably IL-1β at a concentration of 25 ng/ml, TNF-α, preferably TNF-α at a concentration of 50ng/ml, and PGE2, preferably PGE2 at a concentration of 10⁻⁶ M, and incubation, preferably incubation for 48h at 37°C and 5% CO₂.

A preferred embodiment of the method for producing a pharmaceutical composition as described above with respect to the first aspect of the invention, for inducing an immune response in a human or animal subject or patient in the treatment of a pathologic disease or disorder, comprises the following steps:
A.) providing a population of autologous antigen-presenting mature dendritic cells which have been isolated from a population of immune cells, including monocytes, from peripheral blood of the human or animal subject, and subsequently cultured, differentiated and primed (pulsed, loaded) with an antigenic peptide related to a specific pathogenic disease or disorder of which the human or animal subject suffers; the specific antigenic peptide, i.e. epitope preferably is selected based on database information;
B.) providing a population of autologous (generated and expanded) cytotoxic CD8+ T cells isolated from a population of immune cells from peripheral blood of the same human or animal subject, preferably from the same population of immune cells as of which the dendritic cells were derived; wherein the T cells are preferably left to remain in a population of non-adherent immune cells for subsequent activation;
C.) co-culturing the primed autologous antigen-presenting mature dendritic cells with non-adherent immune cells, including the CD8+ T cells to be activated, wherein the non-adherent cells include at least one of the following: T-lymphocytes, B-lymphocytes, platelets, neutrophils, basophils, eosinophils; wherein said non-adherent cells have been isolated from a population of PBMC from peripheral blood of the same human or animal subject, preferably from the same population of PBMC from which the dendritic cells were derived; wherein the primed autologous antigen-presenting mature dendritic cells non-adherent cells are preferably co-cultured with all the non-adherent immune cells derived from the same population of PBMC from peripheral blood of the same human or animal subject; wherein a preferred ratio of non-adherent immune cells to autologous antigen-presenting mature dendritic cells is 30:1; wherein the co-culturing is preferably carried out in a co-culturing medium for 1 week at 37°C and 5% CO₂, wherein the co-culturing medium preferably is supplemented with one or more interleukins that support cell survival and expansion;
D.) isolation of activated CD8+ T cells (CTL) from the non-adhering immune cells by positive selection using CD8-specific magnetic beads (magnetic beads coupled with an anti-human CD8 antibody);
E.) quality control of isolated CTL.

Preferably, step A.) above further comprises, in a first step, the isolation of peripheral blood from the human or animal subject, comprising peripheral blood mononuclear cells (PBMCs) as a starting material for the isolation of the PBMCs.

A further preferred embodiment of the method according to the present invention comprises, in step A.) a step of separating monocytes from the PBMCs. For the purpose of separating intact white blood cells from the red blood cells, red blood cell (RBC) lysis buffer is used. Preferably, in a first step, the population of PBMCs from the peripheral blood are left in culture for 2 hours at 37°C and 5% CO₂, and subsequently, a supernatant is collected after monocyte adhesion. The supernatant contains non-adherent cells, comprising non-adherent immune cells. Preferably, the non-adherent immune cells are cryopreserved for future use in the activation of CTL in step C.). The treatment of monocytes is preferably carried out in the presence of 100 ng/ml granulocyte-macrophage colony-stimulating factor (GM-CSF) and 50ng/ml IL-4 for 7 days. This results in a proliferation of monocytes and a differentiation into (still immature) dendritic cells.

Preferably, step A.) furthermore comprises at least the following steps:
e.) culturing monocytes isolated from PBMCs of the human or animal subject, preferably isolated by Ficoll-separation;
f.) culturing of adhering monocytes, preferably with granulocyte-monocyte colony-stimulating factor and IL-4, preferably in RPMI 1640 Medium with 10% heat-inactivated FBS and 1% glutamine, preferably for 6 days, resulting in a population of differentiated, immature DCs;
g.) priming, i.e. pulsing or loading, of the immature DCs with an antigenic peptide, preferably at day 6 of culture, preferably at a final concentration of 10 µg/ml, preferably for 5h, preferably in the presence of β2 microglobulin, preferably at a final concentration of 3µg/ml of β2 microglobulin;
h.) maturing of the loaded immature dendritic cells with a cytokine cocktail (including IL-6 (10ng/ml), IL-1β (25 ng/ml), TNF-α (50ng/ml), and PGE2 (10⁻⁶ M) and incubation, preferably for 48h at 37°C and 5% CO₂,
i.) and preferably freezing aliquots of the mature loaded dendritic cells for a subsequent step of stimulation/activation of CD8+ T cells, preferably according to step C. above.

In another preferred embodiment of the method according the present invention, in step C.), the activation of CD8+ T cells is performed by three separate steps of activation, preferably at intervals of 7 days. Therein, preferably in each step of activation one aliquot of mature DCs pulsed with the antigenic peptide is used to activate the CD8+ T cells. In a first step, non-adherent immune cells containing the CD8+ T cells to be activated are co-cultivated with a first aliquot of mature DCs pulsed with the antigenic peptide, wherein preferably a ratio of non-adherent immune cells to mature DCs is 30:1 (please provide ranges, e.g. 20:1-50:1, preferably 25:1-35:1, more preferably 30:1). Preferably on day 7 of co-cultivating, a second aliquot of the pulsed mature DCs is added to the non-adherent immune cells containing the CD8+ T cells. Preferably on day 14, a third aliquot of the pulsed mature DCs is added to the non-adherent immune cells. Preferably IL-2 and IL-7 are added in the second and third of activation. Preferably, in each of the second and third step, the antigenic peptide is again added at a final concentration of 10µg/ml, and preferably in each of the second and third step, β2 microglobulin is added again, preferably at a final concentration of 3 µg/ml for the purpose of additional stimulation of the CD8+ T cells, i.e. for boosting the immune response.

In order to determine whether the CTL are sufficiently proliferated and sufficiently cytotoxic for use in triggering an immune response after their activation, the following quality control assays are performed: First, the degree of CTL proliferation is determined, preferably by a CFSE assay (Carboxyfluorescin Diacetate Succinimidyl Ester), preferably after five days of co-culture with DC. Additionally, preferably the level of cytotoxicity of CTL is determined, preferably by an LDH cytotoxicity detection assay.

The present invention furthermore concerns a method for treating a pathologic disease or disorder in a human or animal subject, preferably for the treatment of cancer, especially breast cancer, or of a viral infection. Said method of treatment, which can also be termed "endogenous adoptive T cell therapy", comprises the step of administering to said subject a pharmaceutical composition according to the first aspect of the invention as described above, preferably produced by the method according to the first aspect of the invention as described above. Said pharmaceutical composition comprises a therapeutically effective amount (dosage, concentration) of autologous activated CTL capable of specifically recognizing an antigenic peptide related to said disease or disorder, preferably capable of specifically recognizing a viral antigenic peptide or a tumor antigenic peptide, preferably of a length of at least 9 amino acids. Most preferably, the activated CD8+ CTL contained in said pharmaceutical composition are capable of recognizing an antigenic peptide selected from a group consisting of MUC (79-87) TLAPATEPA peptide, SARS-CoV2 S-protein (84-92) LPFNDGVYF peptide (Seq. ID 2), SARS-CoV2 S-protein (1185-1200) RLNEVAKNLNESLIDL peptide (Seq. ID 3), SARS-CoV2 S-protein (1185-1193) RLNEVAKNL peptide (Seq. ID 4), and SARS-CoV2 S-protein (1192-1200) NLNESLIDL peptide (Seq. ID 5). The pharmaceutical composition preferably is administered via one of the following pathways: intravenously, into a cavity adjacent to a location of a solid tumor, such as into the intraperitoneal cavity, or directly infused into or adjacent to a solid tumor.

The present invention furthermore concerns a pharmaceutical composition for the treatment of a pathologic disorder of a human or animal subject, comprising as an active ingredient a therapeutically effective amount of activated CTL capable of recognizing an antigen specific for said pathologic disorder, preferably capable of recognizing an antigenic peptide selected from a group consisting of MUC (79-87) TLAPATEPA peptide, SARS-CoV2 S-protein (84-92) LPFNDGVYF peptide (Seq. ID 2), SARS-CoV2 S-protein (1185-1200) RLNEVAKNLNESLIDL peptide (Seq. ID 3), SARS-CoV2 S-protein (1185-1193) RLNEVAKNL peptide (Seq. ID 4), and SARS-CoV2 S-protein (1192-1200) NLNESLIDL peptide (Seq. ID 5), said pharmaceutical composition optionally further comprising at least one of the following substances: a pharmaceutically acceptable additive, a carrier, an excipient, a stabilizer, wherein the activated CTL preferably have been obtained by the method disclosed above.

The present invention furthermore concerns, according to the second aspect of the invention, a pharmaceutical composition for the treatment of a pathologic disorder of a human or animal subject, comprising as an active ingredient a therapeutically effective amount of autologous primed dendritic cells, presenting, on their cell surface MHC, an antigenic peptide selected from a group consisting of MUC (79-87) TLAPATEPA peptide, SARS-CoV2 S-protein (84-92) LPFNDGVYF peptide (Seq. ID 2), SARS-CoV2 S-protein (1185-1200) RLNEVAKNLNESLIDL peptide (Seq. ID 3), SARS-CoV2 S-protein (1185-1193) RLNEVAKNL peptide (Seq. ID 4), and SARS-CoV2 S-protein (1192-1200) NLNESLIDL peptide (Seq. ID 5), said pharmaceutical composition optionally further comprising at least one of the following substances: a pharmaceutically acceptable additive, a carrier, an excipient, a stabilizer, wherein the autologous primed dendritic cells have been obtained by the method disclosed above.

The present invention furthermore concerns a pharmaceutical composition as described above, with respect to the first or the second aspect of the invention, for use as a medicament in the treatment of a pathologic disorder of a human or animal subject, preferably in the treatment of cancer or of a viral infection.

The invention further concerns, with respect to the first aspect of the invention, the use of activated autologous CTL in a pharmaceutical composition or a vaccine in an endogenous adoptive T cell therapy for the treatment of a pathologic disease or disorder in a human or animal subject suffering from the pathologic disease, preferably from cancer, such as breast cancer, wherein the activated CD8+ CTL are capable of recognizing an antigenic peptide, preferably of recognizing a MUC (79-87) TLAPATEPA peptide, following the activation of the CD8+ T-cells by autologous dendritic cells primed (loaded) with and presenting said antigenic peptide.

The invention further concerns the use of activated autologous cytotoxic CD8+ CTL in a pharmaceutical composition or a vaccine in an endogenous adoptive T cell therapy for the treatment or prevention of a viral infection, wherein the activated CD8+ CTL are capable of recognizing an antigenic peptide selected from a group consisting of SARS-CoV2 S-protein (84-92) LPFNDGVYF peptide (Seq. ID 2), SARS-CoV2 S-protein (1185-1200) RLNEVAKNLNESLIDL peptide (Seq. ID 3), SARS-CoV2 S-protein (1185-1193) RLNEVAKNL peptide (Seq. ID 4), and SARS-CoV2 S-protein (1192-1200) NLNESLIDL peptide (Seq. ID 5), following the activation of the CD8+ T-cells by autologous dendritic cells primed (loaded) with and presenting said antigenic peptide.

The invention further concerns, with respect to the second aspect of the invention, the use of autologous primed dendritic cells in a pharmaceutical composition or a vaccine in an endogenous adoptive T cell therapy for the treatment of a pathologic disease or disorder in a human or animal subject suffering from the pathologic disease, preferably from cancer, such as breast cancer, wherein the autologous primed dendritic cells each present on their cell surface an antigenic peptide, preferably a MUC (79-87) TLAPATEPA peptide.

The invention further concerns the use of autologous primed dendritic cells in a pharmaceutical composition or a vaccine for the treatment or prevention of a viral infection, wherein the autologous primed dendritic cells each present on their cell surface an antigenic peptide selected from a group consisting of SARS-CoV2 S-protein (84-92) LPFNDGVYF peptide (Seq. ID 2), SARS-CoV2 S-protein (1185-1200) RLNEVAKNLNESLIDL peptide (Seq. ID 3), SARS-CoV2 S-protein (1185-1193) RLNEVAKNL peptide (Seq. ID 4), and SARS-CoV2 S-protein (1192-1200) NLNESLIDL peptide (Seq. ID 5).

Further embodiments of the invention are laid down in the dependent claims.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1: shows an image of phase-contrast microscopy of immature dendritic cells at a magnification of X40;
- Fig. 2: shows an image of phase-contrast microscopy of mature dendritic cells at a magnification of X40;
- Fig. 3: shows an image of phase-contrast microscopy of all non-adherent cells co-cultured with mature dendritic cells at a magnification of X10;
- Fig. 4: shows the results of a CFSE proliferation assay;
- Fig. 5A,B: show the results of an LDH cytotoxicity detection assay, wherein in Fig. 5A, the results are shown in a plate-format and in Fig. 5B, in table format.

### DESCRIPTION OF PREFERRED EMBODIMENTS

The present invention describes, in a first aspect, a novel approach of the development of autogenous antigen specific CTLs that can be administered for adoptive T cell therapy, as well as, in a second aspect, the priming of autogenous dendritic cells to present an antigenic peptide. The cells that are used herein are autologous, i.e. from the same species and same donor, as the recipient subject, i.e the patient suffering from a specific disease or disorder, which may or may not be pathogen related. The first aspect of this invention is the selection of an appropriate 9-mer peptide of the antigen that is related to the specific pathogen or disease of which the human subject, i.e. patient suffers or is at risk to suffer (in case of a vaccine). The selection can be performed from databases that are well known in the art such as the SYFPEITHI MHC databank, an epitope prediction database. The selection is based on comparison of the higher scores of immunogenicity and T cell epitope prediction derived from such databases. The next step is the isolation of immune cells from the peripheral blood. The peripheral blood contains monocytes, T lymphocytes, B lymphocytes, NK cells, platelets, neutrophils, eosinophils, basophils. Characterization of the immune cell populations can be performed with flow cytometry (forward scatter vs. side scatter analysis). DCs which in the method according to the invention serve as the antigen presenting cells, can be obtained from the patient's peripheral blood following protocols known in the art for monocyte isolation and dendritic cell differentiation. Differentiation of monocytes to dendritic cells is achieved in the presence of cytokines well known in the art, such as the granulocyte-macrophage colony-stimulating factor (GM-CSF) and interleukin 4 (IL-4). DCs that have been stimulated with GM-CSF and IL-4 express MHC class I and class II molecules. The synthetic selected antigenic peptide can be obtained from any qualified manufacturer. "Loading" of the dendritic cells with said peptide, a step also termed "priming" or "pulsing", is preferably performed with the addition of β2 microglobulin. This step is followed by the maturation of DCs in the presence of a "cocktail" of cytokines. According to the first aspect of the invention, the autologous primed mature DCs are used for ex vivo activation of autologous CD8+ T cells, i.e. for triggering an immune response ex vivo. As an alternative, according to the second aspect of the invention, these autologous primed mature DCs can either be used directly in a pharmaceutical composition for administration to the human or animal subject for triggering an immune response, i.e. activation of CD8+ T cells in vivo following administration.
The effector cells (CD8+ T cells) used in the method according to the first aspect of the invention can be generated and expanded in vitro in accordance with known techniques (including but not limited to those described in Ranieri, Cytotoxic T-Cells: Methods & Protocols, ISBN: 978-1-4939-1157-8, Humana Press, New York, NY, 2014) or variations thereof that are well known to those skilled in the art.
The activation of CD8+ T cells can be achieved by culturing a population of cells containing CD8+ T cells with an aliquot of mature DCs pulsed with the selected antigenic peptide. Some protocols first select only the CD8+ T lymphocytes from the population of PBMCs (of which the monocytes have already been separated for the purpose of generation of DCs). However, according to a preferred embodiment of the present invention, the mature, pulsed DCs were co-cultured with all the non-adherent cells that have been isolated, including T lymphocytes, B lymphocytes, platelets, neutrophils, basophils, eosinophils. Preferably, in the method according to the present invention, for the purpose of activation of CTLs, the pulsed/loaded DCs were co-cultured with the majority of the immune cells, i.e. all immune cells isolated from the peripheral blood except for the monocytes. The reason for doing so is to mimic the processes in the immune system in vivo, i.e. to stimulate the interactions that physiologically happen in vivo. This leads to a stronger activation and thus a greater degree of cytotoxicity of CTL, as the cell-cell interactions, cell-signaling and differentiation pathways, i.e. reactions to cytokine release from various cells are increased.

The non-adherent cells obtained from peripheral blood, preferably from the same sample as used for the isolation of monocytes, are thawed using protocols known in art, and subsequently co-cultured with the mature DCs preferably for 1 week at 37 °C and 5 % CO₂. Therein, the ratio of non-adherent cells to mature DCs preferably is 30:1.. The medium for the co-cultivation preferably is supplemented with interleukins which support cell survival and expansion. Isolation of activated CD8+ T cells from the bulk T cell cultures is preferably performed by positive selection using magnetic beads coated with an antibody specific for the CD8 membrane antigen. Magnetic beads can be removed from the positively isolated cells using protocols known in art.
After CD8+ T-cell selection, the activated CTLs are ready for quality control, such as proliferation and cytotoxic activity, and when they pass the assessment they can be administered back to the patient. Cells can furthermore be analyzed in terms of gene and protein expression. T cell proliferation can be determined by CFSE assay, where every generation of cells is presented with a different subset in flow cytometry. Thereby, distinct generations of proliferating cells can be monitored by dye dilution. Live cells are covalently labeled with a very bright, stable dye. Every generation of cells appears as a different peak on a flow cytometry histogram. Suitable means for evaluating the biologic activity of the cells may be the in vitro stimulation of CTLs with target cells for a period of time, usually 24 or 48h, and determination of cytokine production by a suitable protein-based detection assay, for example by flow cytometry, ELISPOT or ELISA assay. Moreover, measurement of the cytotoxic activity of CTLs can be performed by in vitro stimulation of CTLs with target cells and determination with suitable means, for example by an LDH cytotoxicity detection assay. Alternatively, the activated CTLs generated according to the method with respect to the first aspect of the invention may be evaluated using an in vivo animal model suitable for the targeting of the CTLs.
The CTLs produced according to the method of the present invention are intended for the induction of an immune response in the recipient human or animal subject. The cells administered to the subject are autologous cells, i.e. the donor is the same as the recipient. The pharmaceutical composition can be prepared by any of the methods well known in the art of pharmacy that are non-toxic to the recipients at the concentrations and dosages used. Preferably, at least 10⁶ activated CTL are used for infusion. Formulations typically require one active ingredient, being the population of activated CTLs, with one or more acceptable carriers. The pharmaceutical composition comprising the activated CTLs can then be directly administered to the subject to be treated. It can be administered intravenously, or into a cavity adjacent to the location of a solid tumor (for example, intraperitoneal cavity) or directly infused into or adjacent to a solid tumor. Sterile injectable solutions well known in the art can be used for resuspension of the CTLs and as carriers for the injection, such as for example 0.9% sodium chloride.

### Example 1:

### CD8+ T cells from a breast cancer patient activated against MUC (79-87) TLAPATEPA (Seq. ID 1) efficiently kill MCF7 human breast adenocarcinoma cells

### Epitope selection:

MUC1 is a single pass type I transmembrane protein with an extracellular domain that is heavily glycosylated and extends up to 200-500nm from the cell surface and it is normally expressed in epithelial cells. Tumor-associated MUC1 is overexpressed and under glycosylated in most human epithelial cancers. It has been considered as a remarkable target for immunotherapies, although so far MUC1 targeting vaccines have not met significant benefits to the patients [Scheikl-Gatard T.,et al., J Transl Med. 15, 154 (2017)].

### Cell isolation:

Peripheral blood mononuclear cells (PBMC) were isolated from the blood of a cancer patient with Ficoll-separation (Biocoll separating solution-Catalog#1077, Biochrom, Berlin, Germany).

### Generation of DCs:

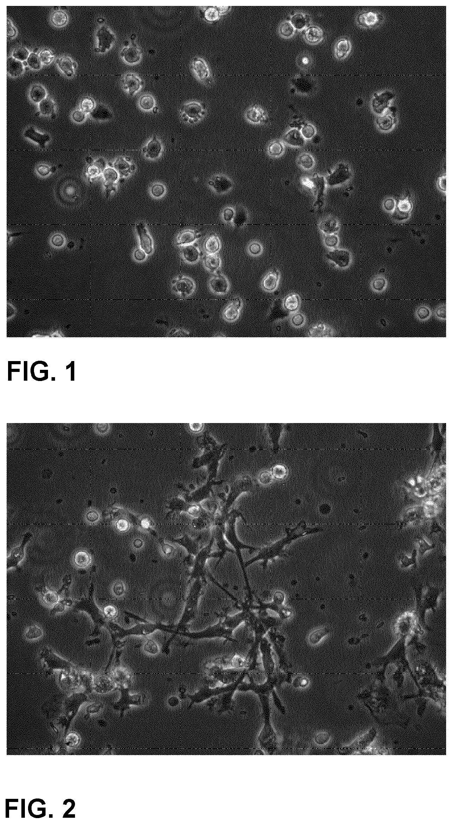

Peripheral blood contains monocytes or immature dendritic cells (DC) that can differentiate in the presence of cytokines. Separation of monocytes from the other types of cells is carried out, as monocytes adhere to the flask during incubation. For this purpose, PBMCs were cultured for 2h at 37°C and 5% CO₂. After monocyte adhesion, the supernatant was collected and non-adherent cells, which included T-lymphocytes, B-lymphocytes, platelets, neutrophils, basophils, and eosinophils, were cryopreserved for later use in the "activation" step. Proliferation of isolated monocytes and differentiation into DC was performed by treatment of the isolated monocytes with 100 ng/ml granulocyte-macrophage colony-stimulating factor (GM-CSF) and 50 ng/ml interleukin 4 (IL-4) for 7 days. This resulted in a population of differentiated, but still immature DC (see figure 1. The generation of immature DCs and the progress of the development of mature DCs was reviewed by phase-contrast inverted microscopy. At the beginning of the culture, monocytes were spherical and adherent to the surface of the flask. At day 5, immature DCs were found forming clusters suspending in culture medium.

### Pulsing of DCs:

Pulsing/priming of immature DCs was carried out with the addition of the MUC(79-87) TLAPATEPA peptide at the final concentration of 10 µg/ml and β2 microglobulin at the final concentration of 3 µg/ml and at incubation time 2-4 h at 37 °C and 5 % CO₂, with occasional agitation. After pulsing of the immature DCs with the peptide, supernatant was removed from the flask, centrifuged, discarded and the cell pellet was resuspended in fresh medium (RPMI 1640) containing IL-1β at 25 ng/ml, TNF-α at 50 ng/ml, IL-6 at 10 ng/ml, PGE2 at 10⁻⁶ M and 20µl Pen/Strep. Cells were left in culture for 48 h at 37 °C and 5 % CO₂ for maturation. Maturation was verified by phase-contrast inverted microscopy. In figure 2, it is shown that upon maturation, DCs were transformed into large irregular cells with cytoplasmic projections. Mature DCs were distributed in three aliquots and two aliquots thereof were cryopreserved for further use.

### Activation and expansion of CTLs:

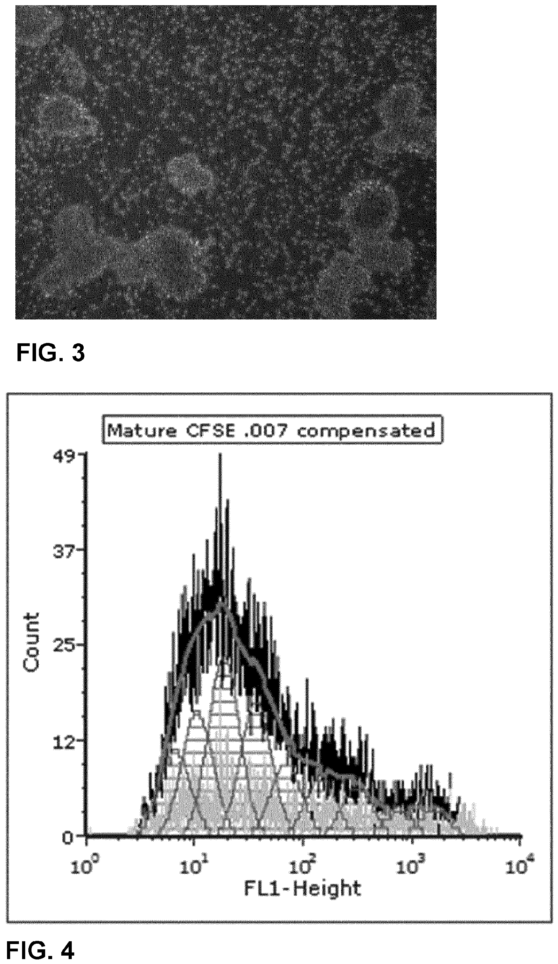

For the activation and expansion of CTLs, an aliquot of non-adherent cells (from the PBMC population of which the adherent monocytes were isolated and removed) were thawed and co-cultivated with an aliquot of mature DCs pulsed with the MUC(79-87) TLAPATEPA peptide in OPTMIZER T CELL EXPANSION SFM containing 1% L-Glutamine, IL-2 at 10ng/ml and 10% Pen/Strep. On day 7, the second aliquot of mature DCs was thawed and the stimulation of T cells was repeated with the addition of IL-2 at 10ng/ml and IL-7 at 5ng/ml, at a final concentration of the MUC(79-87) TLAPATEPA peptide of 10 µg/ml and a final concentration of β2 microglobulin of 3 µg/ml. In figure 3, an image of all non-adherent cells, including T-cells co-cultured with dendritic cells is shown.

On day 14, the third aliquot of mature DCs was thawed and the stimulation of T cells was repeated in the same way as the first and second stimulation with the addition of IL-2 at 10ng/ml and IL-7 at 5ng/ml, at a final concentration of the MUC(79-87) TLAPATEPA peptide of 10 µg/ml and a final concentration of β2 microglobulin of 3 µg/ml..

On day 21, CD8+ T cells were separated and isolated from the bulk T cell cultures by positive selection using magnetic beads specific for CD8.

### CFSE Assay:

T cell proliferation was assessed with CellTraceTM CFSE Cell Proliferation Kit Catalog#C34554 (ThermoFischer Scientific, Darmstadt, Germany) according to the manufacturer's suggestions. After five days of co-culture with DCs, live T cells were analysed for Carboxyfluorescein Diacetate Succinimidyl Ester (CFSE) dilution by flow cytometry. Every generation of cells is presented with a different subset on flow cytometry. As controls, unstimulated T cells cultured alone, showed the CFSE intensity of non-divided cells, while non-labeled cells showed the auto-fluorescence of the cells and the limits of detectable cell divisions. As shown in figure 4, a data set was analysed using the proliferation plot on FCS Express to determine the ability of mature DCs to stimulate T cell proliferation. The software was able to recognize the first generation (undivided cells) and counted a total of 9 generations of divided cells of the CD8+ T cells that had been stimulated with mature DCs that were pulsed with MUC(79-87) TLAPATEPA peptide.

### LDH Cytotoxicity Detection Assay:

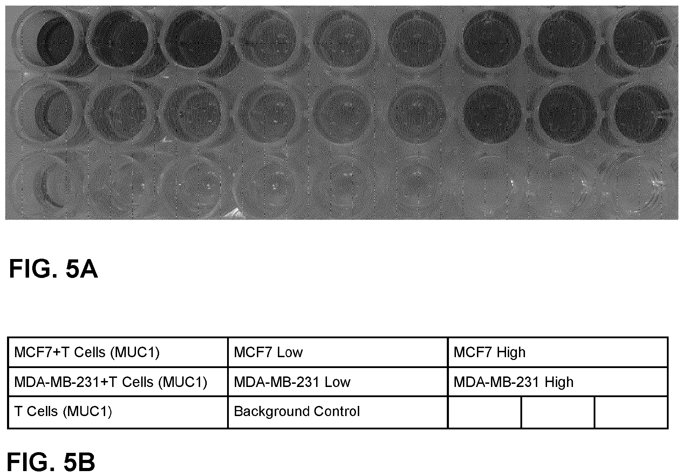

The assay was performed by following the manufacturer's advice. Briefly, 500 cancer cells were seeded in a U-bottom 96-well plate Catalog#4430200 (Orange Scientific, Braine-I'Alleud, Belgium) and were left overnight at 37°C to adhere. On the next day, preactivated T cells were added at a ratio of 1:10 and co-cultured for 20 hr at a final volume of 100 µl. Plates were centrifuged at 2000 rpm for 10 minutes and 50 µl of supernatants were transferred into corresponding wells of an optically clear 96-well flat bottom microplate Catalog#781722 (Brand, Wertheim, Germany). The level of cytotoxicity was measured with Cytotoxicity Detection Kit (LDH) Catalog#11644793001 (Roche, Darmstadt, Germany). 50 µl of substrate were added to the corresponding wells and microplate was left to incubate for 30 minutes at room temperature in the dark. Absorbance was measured at 490 nm using an ELISA reader at a reference wavelength of 605 nm. The percentage of cytotoxicity was calculated as (experimental value- spontaneous effector cell release - spontaneous target cell release) / (maximum target cell release - spontaneous target cell release) × 100%. The background value was subtracted from all the above values. In figure 5A, activated CTLs targeting MCF7 and MDA-MB-231 human breast adenocarcinoma cell lines are shown. The darker color represents higher cell death. As shown in figure 5B, "low" controls represent regular death rate of cells, while "high" controls represent death rate of cells with Triton-X 100. The background Control is RPMI + 1% FBS.

### Results:

The in vitro generation of antigen-specific CTL from a breast patient's blood was successfully achieved. The process required careful handling of the samples as primary cells were very sensitive. It seems that activation triggered the CD8+ T cells to start proliferating as there were nine generations of new T cells. Results from the LDH Cytotoxicity Detection Assay demonstrated that MUC1-specific CTLs had the ability to induce cytotoxicity in human breast adenocarcinoma cell lines. MCF7 and MDA-MB-231 were two cell lines that had different characteristics, in that MCF7 was a population from a patient suffering from luminal type breast cancer and MDA-MB-231 was a population from a patient suffering from triple negative breast cancer. The two cell lines resulted in different resistance to CTLs. CTLs which had been activated to recognize the MUC(79-87) TLAPATEPA peptide, demonstrated greater cytotoxic effect in the MCF7 cell line (106%) than in the MDA-MB-231 cell line (9,8%). The percentage of cytotoxicity was calculated as (experimental value - spontaneous effector cell release - spontaneous target cell release) / (maximum target cell release - spontaneous target cell release) x 100%. The background value was subtracted from all the above values. A value above 100% is to be interpreted as a very high cytotoxicity, i.e. the whole population of target cells was killed. The absorbance coming from the sample in this colorimetric assay was higher than the "high" control. These results suggest that the activated CTLs killed almost the whole population of MCF7 target cells.

### Example 2:

### CD8+ T cells from healthy donors activated against SARS-CoV2 S-protein (84-92) peptide LPFNDGVYF (Seq. ID 2)

### Epitope selection:

Similar to other coronaviruses, the spike (S) protein of SARS-CoV, the severe acute respiratory syndrome coronavirus 2, is a large type I transmembrane glycoprotein with multiple biological functions. The predicted S1 subunit corresponding to the region of amino acids (aa) 13 to 680 contains the minimal receptor-binding domain (RBD) and mediates binding of the S protein to angiotensin-converting enzyme 2 (ACE2), a functional receptor on susceptible cells. The predicted S2 subunit (aa 681 to 1255) contains two heptad repeat regions (HR1 and HR2) and is responsible for fusion between viral and cellular membranes. A second major feature of coronavirus S protein is its capacity to induce neutralizing antibodies and protective immunity, and it is thereby considered a major target for vaccine development (He et al., 2006).

In example 2, immature dendritic cells are primed with SARS-CoV2 S-protein (84-92) peptide LPFNDGVYF (Seq. ID 2), a peptide of the surface glycoprotein, the so-called "spike protein" of SARS-CoV2 and then matured. The primed DCs were then either co-cultured with non-adherent immune cells including CD8+ T cells as described in example 1 for inducing an immune response ex vivo, or directly used in a pharmaceutical composition for triggering an immune response in vivo. -The activation of T cells can be verified by extracting a blood sample from the subjects and confirming the presence of activated CTLs against the specific antigen.

### Example 3:

### CD8+ T cells from human patients or from healthy donors, respectively, activated ex vivo or in vivo against SARS-CoV2 S-protein (1185-1200) RLNEVAKNLNESLIDL (Seq. ID 3)

### Epitope selection:

In example 3, immature dendritic cells are also primed with a SARS-CoV2 S-protein peptide of the surface glycoprotein, the so-called "spike protein", i.e. S-protein of SARS-CoV2, as in example 2. However, this time with SARS-CoV2 S-protein (1185-1200) peptide RLNEVAKNLNESLIDL (Seq. ID 3) and then matured. This antigentic peptide of the SARS-CoV-2 has a length of 16 amino acids. The recognition also happens at a sequence of nine amino acids but the full length peptide is more immunogenic. The primed DCs can then either be co-cultured with non-adherent immune cells including CD8+ T cells as described in example 1 for inducing an immune response ex vivo, or directly used in a pharmaceutical composition for triggering an immune response in vivo. - The activation of T cells can be verified by extracting a blood sample from the subjects and confirming the presence of activated CTLs against the specific antigen.

### Example 4:

### CD8+ T cells from human patients or from healthy donors, respectively, activated ex vivo or in vivo against SARS-CoV2 S-protein (1185-1193) RLNEVAKNL (Seq. ID 4)

In example 4, immature dendritic cells are primed with SARS-CoV2 S-protein (1185-1193) RLNEVAKNL (Seq. ID 4), a peptide of the surface glycoprotein, the so-called "spike protein" of SARS-CoV2 and then matured. The primed DCs can then either be co-cultured with non-adherent immune cells including CD8+ T cells as described in example 1 for inducing an immune response ex vivo, or directly used in a pharmaceutical composition for triggering an immune response in vivo. The activation of T cells can be verified by extracting a blood sample from the treated subjects and confirming the presence of activated CTLs against the specific antigen.

### Example 5:

### CD8+ T cells from human patients or from healthy donors, respectively, activated ex vivo or in vivo against SARS-CoV2 S-protein (1192-1200) NLNESLIDL (Seq. ID 5)

In example 5, immature dendritic cells are primed with SARS-CoV2 S-protein (1192-1200) NLNESLIDL (Seq. ID 5), a peptide of the surface glycoprotein, the so-called "spike protein" of SARS-CoV2 and then matured. The primed DCs can then either be co-cultured with non-adherent immune cells including CD8+ T cells as described in example 1 for inducing an immune response ex vivo, or directly used in a pharmaceutical composition for triggering an immune response in vivo. -The activation of T cells can be verified by extracting a blood sample from the subjects and confirming the presence of activated CTLs against the specific antigen.

### LIST OF REFERENCE SIGNS

none

### REFERENCES

Eisenbarth, S.C. Dendritic cell subsets in T cell programming: location dictates function. Nat Rev Immunol 19, 89-103 (2019). https://doi.org/10.1038/s41577-018-0088-1
Farhood, B, Najafi, M, Mortezaee, K. CD8+ cytotoxic T lymphocytes in cancer immunotherapy: A review. J Cell Physiol. 2019; 234: 8509-8521. https://doi.org/10.1002/jcp.27782.
Franck J. Barrat, Lishan Su; A pathogenic role of plasmacytoid dendritic cells in autoimmunity and chronic viral infection. J Exp Med 2 September 2019; 216 (9): 1974-1985. doi: https://doi.org/10.1084/jem.20181359.
He Y, Li J, Heck S, Lustigman S, Jiang S. Antigenic and immunogenic characterization of recombinant baculovirus-expressed severe acute respiratory syndrome coronavirus spike protein: implication for vaccine design. J Virol. 2006;80(12):5757-5767. doi:10.1128/JVI.00083-06.
Jebastin, T., Narayanan, S., In silico epitope identification of unique multidrug resistance proteins from Salmonella Typhi for vaccine development, Comput. Biol. Chem., 2019 Feb; 78:74-80, ISSN 1476-9271, https://doi.org/10.1016/i.compbiolchem.2018.11.020.
Krause P.J., Kavathas P.B., Ruddle N.H. (2019) Immunotherapy for Infectious Diseases, Cancer, and Autoimmunity. In: Krause P., Kavathas P., Ruddle N. (eds) Immunoepidemiology. Springer, Cham
Maddur, M.S., Lacroix-Desmazes, S., Dimitrov, J.D. et al. Natural Antibodies: from First-Line Defense Against Pathogens to Perpetual Immune Homeostasis. Clinic Rev Allerg Immunol 58, 213-228 (2020). https://doi.org/10.1007/s12016-019-08746-9.
Met, Ö., Jensen, K.M., Chamberlain, C.A. et al. Principles of adoptive T cell therapy in cancer. Semin Immunopathol 41, 49-58 (2019). https://doi.org/10.1007/s00281-018-0703-Z.
Roy A. Mariuzza, Pragati Agnihotri and John Orban (2020) The structural basis of T-cell receptor (TCR) activation: An enduring enigma J. Biol. Chem. 2020, 295:914-925 doi: 10.1074/jbc.REV119.009411 originally published online December 17, 2019
Sant, A.J., 6 - Overview of T-Cell Recognition: Making Pathogens Visible to the Immune System, Editor(s): Robert R. Rich, Thomas A. Fleisher, William T. Shearer, Harry W. Schroeder, Anthony J. Frew, Cornelia M. Weyand, Clinical Immunology (Fifth Edition), Content Repository Only!, 2019, Pages 93-106.e1, ISBN 9780702068966, https://doi.org/10.1016/B978-0-7020-6896-6.00006-5.

## Claims

1. A pharmaceutical composition for inducing an immune response in a human or animal subject suffering from a pathologic disease or disorder, comprising autologous activated cytotoxic CD8+ T-lymphocytes (CTL), activated by autologous primed mature dendritic cells of the human or animal subject, said autologous primed mature dendritic cells presenting an antigenic peptide,
- wherein the activated CTL are derived from an autologous population of peripheral blood mononuclear cells (PBMCs) isolated from peripheral blood of the same human or animal subject; and
- wherein the activated CTL are able to recognize the antigenic peptide;
- wherein the CTL have been activated by autologous dendritic cells primed with an antigenic peptide, wherein the dendritic cells have been isolated from the same human or animal subject as the CTL, wherein the CTL have been activated by co-culturing CD8+ T-cells derived from the population of PBMC, with the antigen-presenting dendritic cells;
- and wherein the dendritic cells prior to their use in the activation of CD8+ T cells have been cultured ex vivo and subsequently have been primed (loaded, pulsed) with the antigenic peptide, and have been matured in the presence of a cytokine cocktail prior to their use in activation of the CD8+ T cells by co-culturing.

2. Pharmaceutical composition of claim 1, wherein the antigenic peptide is a tumor antigenic peptide, preferably a MUC1-peptide, more preferably of a length of at least 9 amino acids, preferably of exactly 9 amino acids, and most preferably a MUC (79-87) TLAPATEPA peptide (Seq. ID 1).

3. Pharmaceutical composition of claim 1, wherein the antigenic peptide is a viral antigenic peptide, preferably a peptide of SARS-CoV2 S-protein, more preferably selected from the group consisting of SARS-CoV2 S-protein (84-92) LPFNDGVYF peptide (Seq. ID 2), SARS-CoV2 S-protein (1185-1200) RLNEVAKNLNESLIDL peptide (Seq. ID 3), SARS-CoV2 S-protein (1185-1193) RLNEVAKNL peptide (Seq. ID 4), and SARS-CoV2 S-protein (1192-1200) NLNESLIDL peptide (Seq. ID 5).

4. Pharmaceutical composition according to one of the preceding claims, wherein the CTL have been activated by co-culturing with the loaded mature dendritic cells a population of all non-adhering immune cells derived from the same population of PBMC as the population of which the dendritic cells have been derived, said population of non-adherent immune cells including the CD8+ T cells to be activated, as well as at least one of the following group of cells: CD4+ T-lymphocytes, B lymphocytes, platelets, neutrophils, basophils and eosinophils.

5. A method for obtaining human or animal autologous dendritic cells presenting an antigenic peptide, preferably a tumor antigenic peptide or viral antigenic peptide of a length of at least 9 amino acids, more preferably selected from the following group consisting of MUC(79-87) TLAPATEPA peptide, SARS-CoV2 S-protein (84-92) LPFNDGVYF peptide (Seq. ID 2), SARS-CoV2 S-protein (1185-1200) RLNEVAKNLNESLIDL peptide (Seq. ID 3), SARS-CoV2 S-protein (1185-1193) RLNEVAKNL peptide (Seq. ID 4), and SARS-CoV2 S-protein (1192-1200) NLNESLIDL peptide (Seq. ID 5) for the preparation of a pharmaceutical composition according to one of claims 1-3, comprising the following steps:
a.) culturing monocytes isolated from PBMCs of the human or animal subject suffering from a pathologic disease or disorder, said monocytes preferably isolated with Ficoll-separation;
b.) culturing of adhering monocytes of step a.) with granulocyte-monocyte colony-stimulating factor and IL-4, preferably in RPMI 1640 Medium with 10% heat-inactivated FBS and 1% glutamine, preferably for 6 days, resulting in a population of immature dendritic cells;
c.) pulsing of the immature dendritic cells of step b.), preferably at day 6 of culture with the antigenic peptide, preferably at a final concentration of 10 µg/ml, and incubation, preferably for 5h, preferably in the presence of β2 microglobulin, preferably at a final concentration of 3µg/ml of β2 microglobulin, resulting in a population of loaded dendritic cells presenting the antigenic peptide;
d.) maturing of the loaded dendritic cells presenting the antigenic peptide of step c.) with a cytokine cocktail, preferably including IL-6, preferably IL-6 at a concentration of 10ng/ml, IL-1β, preferably IL-1β at a concentration of 25 ng/ml, TNF-α, preferably TNF-α at a concentration of 50ng/ml, and PGE2, preferably PGE2 at a concentration of 10⁻⁶ M, and incubation, preferably incubation for 48h at 37°C and 5% CO₂.

6. A method for producing a pharmaceutical composition, preferably a pharmaceutical composition according to one of the preceding claims, for inducing an immune response in a human or animal subject in the treatment of a pathologic disease, comprising the following steps:
A.) providing a population of autologous antigen-presenting mature dendritic cells which have been isolated from a population of immune cells, including monocytes, from peripheral blood of the human or animal subject, and subsequently cultured, differentiated and primed with an antigenic peptide related to a specific pathogenic disease or disorder of which the human or animal subject suffers;
B.) providing a population of autologous (generated and expanded) cytotoxic CD8+ T cells isolated from a population of immune cells from peripheral blood of the same human or animal subject, preferably from the same population of immune cells as of which the dendritic cells were derived;
C.) co-culturing the primed autologous antigen-presenting mature dendritic cells with non-adherent immune cells, including the CD8+ T cells to be activated, wherein the non-adherent cells include at least one of the following: T-lymphocytes, B-lymphocytes, platelets, neutrophils, basophils, eosinophils; wherein said non-adherent cells have been isolated from a population of PBMCs from peripheral blood of the same human or animal subject, preferably from the same population of PBMCs from which the dendritic cells were derived; wherein the primed autologous antigen-presenting mature dendritic cells non-adherent cells are preferably co-cultured with all the non-adherent immune cells derived from the same population of PBMCs from peripheral blood of the same human or animal subject; wherein a preferred ratio of non-adherent immune cells to autologous antigen-presenting mature dendritic cells is 30:1; wherein the co-culturing is preferably carried out in a co-culturing medium for 1 week at 37°C and 5% CO₂, wherein the co-culturing medium preferably is supplemented with one or more interleukins that support cell survival and expansion;
D.) isolation of CD8+ CTLs from the non-adhering immune cells by positive selection using CD8-specific magnetic beads;
E.) quality control of isolated CD8+ CTLs.

7. Method according to claim 6, wherein step A.) comprises isolation of peripheral blood from the human or animal subject, comprising PBMCs as a starting material for the isolation of the PBMCs.

8. Method according to claim 6, wherein step A.) comprises a step of separating monocytes from the PBMC, wherein preferably, in a first step, PBMCs are left in culture for 2 hours at 37°C and 5% CO₂, and subsequently, collection of a supernatant after monocyte adhesion, and preferably cryopreservation of non-adherent cells for future use in activation of CD8+ T cells in step C.), and preferably treatment of monocytes with 100 ng/ml granulocyte-macrophage colony-stimulating factor (GM-CSF) and 50ng/ml IL-4 for 7 days for proliferation of monocytes and differentiation into dendritic cells.

9. Method according to claim 6, wherein step A.) comprises at least the following steps:
e.) culturing monocytes isolated from peripheral blood mononuclear cells of the human or animal subject, preferably isolated by Ficoll-separation;
f.) culturing of adhering monocytes, preferably with granulocyte-monocyte colony-stimulating factor and IL-4, preferably in RPMI 1640 Medium with 10% heat-inactivated FBS and 1% glutamine, preferably for 6 days, resulting in a population of differentiated, immature dendritic cells;
g.) priming of the immature DCs with an antigenic peptide, preferably at day 6 of culture, preferably at a final concentration of 10 µg/ml, preferably for 5h, preferably in the presence of β2 microglobulin, preferably at a final concentration of 3µg/ml of β2 microglobulin;
h.) maturing of the loaded (primed) immature dendritic cells with a cytokine cocktail, preferably including IL-6, IL-1β, TNF-α, and PGE2 and incubation, preferably for 48h at 37°C and 5% CO₂,
i.) and preferably freezing aliquots of the mature loaded dendritic cells for a subsequent step of stimulation of CD8+ T cells.

10. Method according to claim 6, wherein in step C.), the activation of CD8+ T cells is performed by three separate steps of activation, preferably at intervals of 7 days, wherein preferably in each step of activation one aliquot of mature DCs pulsed with the antigenic peptide is used to activate the CD8+ T cells, wherein preferably in a first step, non-adherent immune cells containing CD8+ T cells are co-cultivated with a first aliquot of mature DCs pulsed with the antigenic peptide, wherein preferably a ratio of non-adherent immune cells to mature DCs is 30:1; and wherein preferably on day 7 of co-cultivating, a second aliquot of the pulsed mature DCs is added to the non-adherent immune cells, and wherein preferably on day 14, a third aliquot of the pulsed mature DCs is added to the non-adherent immune cells, wherein preferably in each of the second and third step of activation IL-2 and IL-7 are added, and wherein preferably, in each of the second and third step of activation the antigenic peptide is added at a final concentration of 10µg/ml, and wherein in each of the second and third step of activation preferably β2 microglobulin is added, preferably at a final concentration of 3 µg/ml.

11. Method according to claim 6, wherein the method comprises at least one of the following steps:
- determination of CTL proliferation, preferably by a CFSE assay (Carboxyfluorescin Diacetate Succinimidyl Ester), preferably after five days of co-culture with DC;
- determination of cytotoxicity of CTLs, preferably by an LDH cytotoxicity detection assay.

12. Method of treatment of a pathologic disease or disorder in a human or animal subject, preferably for the treatment of cancer or of a viral infection, comprising the step of administering to said subject a pharmaceutical composition according to one of claims 1-4, preferably produced by the method according to one of claims 6-11, said pharmaceutical composition comprising a therapeutically effective amount (dosage, concentration) of autologous activated CTLs capable of specifically recognizing an antigenic peptide related to said disease or disorder, preferably capable of specifically recognizing a viral antigenic peptide or a tumor antigenic peptide of a length of at least 9 amino acids, most preferably of recognizing an antigenic peptide selected from a group consisting of MUC (79-87) TLAPATEPA peptide, SARS-CoV2 S-protein (84-92) LPFNDGVYF peptide (Seq. ID 2), SARS-CoV2 S-protein (1185-1200) RLNEVAKNLNESLIDL peptide (Seq. ID 3), SARS-CoV2 S-protein (1185-1193) RLNEVAKNL peptide (Seq. ID 4), and SARS-CoV2 S-protein (1192-1200) NLNESLIDL peptide (Seq. ID 5), wherein the pharmaceutical composition is preferably administered via one of the following pathways: intravenously; into a cavity adjacent to a location of a solid tumor, such as into the intraperitoneal cavity; directly infused into or adjacent to a solid tumor.

13. Method of treatment of cancer, preferably of breast cancer, in a human or animal subject, comprising the step of administering to said subject a pharmaceutical composition comprising a therapeutically effective amount of autologous activated CTL capable of specifically recognizing an antigenic peptide selected from a group consisting of MUC (79-87) TLAPATEPA peptide, preferably contained in a pharmaceutical composition produced by the method according to one of claims 6-11.

14. A pharmaceutical composition for use as a medicament in the treatment of a pathologic disorder of a human or animal subject, comprising as an active ingredient a therapeutically effective amount of activated cytotoxic CTLs capable of recognizing an antigen specific for said pathologic disorder, preferably capable of recognizing an antigenic peptide selected from a group consisting of MUC (79-87) TLAPATEPA peptide, SARS-CoV2 S-protein (84-92) LPFNDGVYF peptide (Seq. ID 2), and SARS-CoV2 S-protein (1185-1200) RLNEVAKNLNESLIDL peptide (Seq. ID 3), SARS-CoV2 S-protein (1185-1193) RLNEVAKNL peptide (Seq. ID 4), and SARS-CoV2 S-protein (1192-1200) NLNESLIDL peptide (Seq. ID 5), said pharmaceutical composition optionally further comprising at least one of the following substances: a pharmaceutically acceptable additive, a carrier, an excipient, a stabilizer, wherein the activated CTLs preferably have been obtained by the method defined in one of claims 6-11.

15. Pharmaceutical composition according to one of claims 1-4, for use as a medicament in the treatment of a pathologic disorder of a human or animal subject, preferably in the treatment of cancer or of a viral infection.

16. Use of activated autologous CTLs in a pharmaceutical composition or a vaccine for the treatment of a pathologic disease or disorder in a human or animal subject suffering from the pathologic disease, preferably from cancer or from a viral infection, wherein the activated CTLs are capable of recognizing an antigenic peptide selected from a group consisting of MUC (79-87) TLAPATEPA peptide, SARS-CoV2 S-protein (84-92) LPFNDGVYF peptide (Seq. ID 2), SARS-CoV2 S-protein (1185-1200) RLNEVAKNLNESLIDL peptide (Seq. ID 3), SARS-CoV2 S-protein (1185-1193) RLNEVAKNL peptide (Seq. ID 4), and SARS-CoV2 S-protein (1192-1200) NLNESLIDL peptide (Seq. ID 5), following the activation of CD8+ T-cells by autologous dendritic cells primed with and presenting said antigenic peptide.

17. Pharmaceutical composition, preferably prepared by a method according to claim 5, for use as a medicament in the treatment of a pathologic disorder of a human or animal subject or as a vaccine for the prevention of a pathologic disorder of a human or animal subject, wherein the pathologic disorder preferably is cancer, preferably breast cancer, wherein the pharmaceutical composition comprises a therapeutically effective dosage of autologous primed dendritic cells each presenting on their cell surface an antigenic peptide, preferably a tumor antigenic peptide, more preferably a MUC1 peptide, most preferably a MUC (79-87) TLAPATEPA peptide.

18. Pharmaceutical composition, preferably prepared by a method according to claim 5, for use as a medicament in the treatment of a pathologic disease or disorder of a human or animal subject or as a vaccine for the prevention of a pathologic disease or disorder of a human or animal subject, wherein the pathologic disease preferably is a viral infection, wherein the pharmaceutical composition comprises, a therapeutically effective dosage of autologous primed dendritic cells each presenting on their cell surface a viral antigenic peptide, preferably an antigenic peptide selected from a group consisting of SARS-CoV2 S-protein (84-92) LPFNDGVYF peptide (Seq. ID 2), SARS-CoV2 S-protein (1185-1200) RLNEVAKNLNESLIDL peptide (Seq. ID 3), SARS-CoV2 S-protein (1185-1193) RLNEVAKNL peptide (Seq. ID 4), and SARS-CoV2 S-protein (1192-1200) NLNESLIDL peptide (Seq. ID 5).

19. Use of autologous primed dendritic cells in a pharmaceutical composition or a vaccine for the treatment of a pathologic disease or disorder in a human or animal subject suffering from the pathologic disease, preferably from cancer, such as breast cancer, wherein the autologous primed dendritic cells each present on their cell surface an antigenic peptide, preferably a MUC1 peptide, more preferably a MUC (79-87) TLAPATEPA peptide.

20. Use of autologous primed dendritic cells in a pharmaceutical composition or a vaccine for the treatment or prevention of a viral infection in a human or animal subject, wherein the autologous primed dendritic cells each present on their cell surface an antigenic peptide selected from a group consisting of SARS-CoV2 S-protein (84-92) LPFNDGVYF peptide (Seq. ID 2), SARS-CoV2 S-protein (1185-1200) RLNEVAKNLNESLIDL peptide (Seq. ID 3), SARS-CoV2 S-protein (1185-1193) RLNEVAKNL peptide (Seq. ID 4), and SARS-CoV2 S-protein (1192-1200) NLNESLIDL peptide (Seq. ID 5).


SEQUENCE LISTING

<110> R.G.C.C. Holdings AG

<120> Antigenic Peptides for activation of CTL

<130> F06304

<160> 5

<170> BiSSAP 1.3.6

<210> 1
<211> 9
<212> PRT
<213> Homo sapiens

<220>
<223> synthetic peptide HLA-A*02:01-binding peptide MUC1 (79-87)
      TLAPATEPA

<400> 1
Thr Leu Ala Pro Ala Thr Glu Pro Ala
1               5

<210> 2
<211> 9
<212> PRT
<213> SARS coronavirus

<400> 2
Leu Pro Phe Asn Asp Gly Val Tyr Phe
1               5

<210> 3
<211> 16
<212> PRT
<213> SARS coronavirus

<400> 3
Arg Leu Asn Glu Val Ala Lys Asn Leu Asn Glu Ser Leu Ile Asp Leu
1               5                   10                  15

<210> 4
<211> 9
<212> PRT
<213> Coronaviridae

<400> 4
Arg Leu Asn Glu Val Ala Lys Asn Leu
1               5

<210> 5
<211> 9
<212> PRT
<213> Coronaviridae

<400> 5
Asn Leu Asn Glu Ser Leu Ile Asp Leu
1               5

SEQUENCE LISTING

<110> R.G.C.C. Holdings AG

<120> Antigenic Peptides for activation of CTL

<130> F06304

<160> 5

<170> BiSSAP 1.3.6

<210> 1
<211> 9
<212> PRT
<213> Homo sapiens


<220>
<223> synthetic peptide HLA-A*02:01-binding peptide MUC1 (79-87)
      TLAPATEPA

<400> 1
Thr Leu Ala Pro Ala Thr Glu Pro Ala
1               5

<210> 2
<211> 9
<212> PRT
<213> SARS coronavirus


<400> 2
Leu Pro Phe Asn Asp Gly Val Tyr Phe
1               5

<210> 3
<211> 16
<212> PRT
<213> SARS coronavirus


<400> 3
Arg Leu Asn Glu Val Ala Lys Asn Leu Asn Glu Ser Leu Ile Asp Leu
1               5                   10                  15



<210> 4
<211> 9
<212> PRT
<213> Coronaviridae


<400> 4
Arg Leu Asn Glu Val Ala Lys Asn Leu
1               5

<210> 5
<211> 9
<212> PRT
<213> Coronaviridae

<400> 5
Asn Leu Asn Glu Ser Leu Ile Asp Leu
1               5